# EUROPÄISCHE PATENTSCHRIFT

(11) **EP 4 014 896 B1**
(45) Veröffentlichungstag und Bekanntmachung des Hinweises auf die Patenterteilung: **18.10.2023**
(21) Anmeldenummer: 21212830.0
(22) Anmeldetag: 07.12.2021
(51) Int. Cl.: A61B 17/16, A61B 17/32

(54) **CHIRURGISCHES INSTRUMENT ZUR ENTNAHME VON KNORPELGEWEBE UND/ODER KNOCHENMATERIAL**
SURGICAL INSTRUMENT FOR TAKING CARTILAGE TISSUE AND / OR BONE MATERIAL
INSTRUMENT CHIRURGICAL DESTINÉ À L'ENLÈVEMENT DU TISSU CARTILAGINEUX ET/OU DE LA MATIÈRE OSSEUSE

(30) Priorität: 15.12.2020 DE 102020133579
(43) Veröffentlichungstag der Anmeldung: 22.06.2022
(73) Patentinhaber: Eberle GmbH & Co. KG, 75449 Wurmberg (DE)
(72) Erfinder: EBERLE, Martin, 75449 Wurmberg (DE)
(74) Vertreter: Hoefer & Partner Patentanwälte mbB

(56) Entgegenhaltungen:
- EP-A1- 1 882 455
- WO-A1-95/10981
- WO-A1-98/49945
- US-A- 5 489 291
- US-A1- 2013 274 751

## Beschreibung

Die vorliegende Erfindung betrifft ein rotierbares, chirurgisches Instrument zur Entnahme von Knorpelgewebe oder Knochenmaterial.

Rotierbare chirurgische Instrumente sind aus dem Stand der Technik in unterschiedlichen Ausgestaltungen bekannt. Zur Entnahme von Knorpelgewebe oder Knochenmaterial, nachfolgend als festes Gewebe bezeichnet, werden im Unterschied zu weichem Gewebe üblicherweise Fräser verwendet, welche an einem distalen Ende einen Fräserkopf mit einer Vielzahl von Schneiden aufweisen. Der Fräser kann kugelförmig oder länglich ausgebildet sein und ist an einem hohlen Schaft angeordnet. In Richtung zum proximalen Ende des chirurgischen Instruments ist dann eine Öffnung vorgesehen, welche eine Verbindung mit dem hohlen Schaft bereitstellt. Ein derartiges Instrument ist beispielsweise aus der DE 697 32 580 T2 bekannt. Durch die Verwendung eines derartigen chirurgischen Instruments wird das abzutragende feste Gewebe allerdings in kleinsten Stücken abgeschnitten, so dass im Extremfall ein Gewebebrei entsteht, in welchem eine Vielzahl der Zellen abgestorben sind. Durch den Transport des abgeschnittenen festen Gewebes in den Nuten derartiger Fräser in Richtung zur Öffnung zum hohlen Schaft wird das abgetrennte feste Gewebe weiter zerkleinert. Da beispielsweise derartiges abgetrenntes Knorpelgewebe nach der Entnahme nachgezüchtet werden soll bzw. einem Patienten an einer anderen Stelle direkt wieder injiziert werden soll, ist ein hoher Anteil an abgestorbenem Gewebe schlecht für die gewünschte Reproduktion durch das entnommene feste Gewebe. Die US 2013/0274751 A1 beschreibt ein chirurgisches Instrument nach dem Oberbegriff des Anspruch 1.

Es ist daher Aufgabe der vorliegenden Erfindung, ein chirurgisches Instrument zur Entnahme von Knorpelgewebe und/oder Knochenmaterial bereitzustellen, welches bei einfachem Aufbau und einfacher, kostengünstiger Herstellbarkeit eine Entnahme mit einem möglichst hohen Anteil an lebenden Zellen des festen Gewebes ermöglicht.

Diese Aufgabe wird durch ein chirurgisches Instrument mit den Merkmalen des Anspruchs 1 gelöst. Die Unteransprüche zeigen bevorzugte Weiterbildungen der Erfindung.

Das chirurgische Instrument zur Entnahme von Knorpelgewebe und/oder Knochenmaterial mit den Merkmalen des Anspruchs 1 weißt demgegenüber den Vorteil auf, dass auf sichere und einfache Weise eine Vielzahl von lebenden Zellen von Knorpelgewebe und/oder Knochenmaterial entnommen werden können. Dabei ermöglicht das erfindungsgemäße chirurgische Instrument, dass größere Materialbruchstücke aus dem Material entnommen werden können. Dadurch wird sichergestellt, dass ausreichend lebende Zellen in dem entnommenen Material vorhanden sind. Weiterhin weist das chirurgische Instrument einen besonders ruhigen und vibrationsarmen Lauf auf. Dadurch wird bei der Entnahme von Material sichergestellt, dass nicht andere Bereiche, welche die Entnahmestelle umgeben, durch die Schneiden des chirurgischen Instruments beschädigt werden. Ein weiterer großer Vorteil des erfindungsgemäßen chirurgischen Instruments liegt darin, dass es möglich ist, dass das vom Knorpelgewebe oder vom Knochen abgetrennte Material vollständig aus dem Entnahmebereich herausgeführt werden kann. Dies wird insbesondere dadurch erreicht, dass das chirurgische Instrument eine Schneidenspitze mit einem kuppelförmigen, schneidenfreien Ende am distalen Ende eines Schafts aufweist. Der Schaft ist hohlzylindrisch mit einem Hohlbereich ausgebildet und weist an einem proximalen Ende, welches dem distalen Ende des Schafts entgegengesetzt ist, einen Anschluss für einen Antrieb, insbesondere einen motorischen Antrieb, auf. Abgetrenntes Material kann dabei durch eine Vielzahl von Öffnungen, welche in der Schneidenspitze ausgebildet sind und voneinander durch Stege getrennt sind, vollständig in den Hohlbereich des Schafts eingesaugt werden. Jede der Öffnungen in der Schneidenspitze weist an wenigstens einem durch die Stege gebildeten Rand der Öffnung, vorzugsweise an zwei einander gegenüberliegenden Rändern der Öffnung, eine Schneide auf. Die Schneiden an den Öffnungen reichen somit nicht bis zu einem äußersten distalen Bereich des chirurgischen Instruments, welcher durch das kuppelförmige, schneidenfreie Ende gebildet wird. Die Schneiden reichen allerdings nahe an das eigentliche distale Ende des Instruments, so dass die Schneiden fast bis zum äußersten distalen Ende der Schneidenspitze reichen, jedoch trotzdem ein kuppelförmiges, schneidenfreies, distales Ende gebildet ist. Ferner erweitern sich die Öffnungen in Richtung zur Mittelachse des chirurgischen Instruments. Dadurch wird der Ansaugvorgang des abgetrennten Materials weiter verbessert.

Vorzugsweise ist am Rand jeder Öffnung eine vollständig umlaufende Schneide ausgebildet.

Gemäß einer weiteren bevorzugten Ausgestaltung der Erfindung weist jede Öffnung einen ersten und einen zweiten geradlinigen Schneidenabschnitt und einen bogenförmigen, distalen Schneidenabschnitt auf. Der bogenförmige distale Schneidenabschnitt ist nahe dem kuppelförmigen Ende angeordnet und verbindet die beiden geradlinigen Schneidenabschnitte miteinander. Die beiden geradlinigen Schneidenabschnitte bilden somit die Seiten der Öffnung, so dass das chirurgische Instrument sowohl in einem Rechtslauf als auch einem Linkslauf betrieben werden kann.

Vorzugsweise weist jede Öffnung einen zum proximalen Ende des chirurgischen Instruments gerichteten Basisabschnitt und einen ersten und zweiten bogenförmigen proximalen Schneidenbereich auf. Der erste und zweite proximale Schneidenbereich verbindet dabei die geradlinigen Schneidenabschnitte mit dem Basisabschnitt. Somit ist die Öffnung an jedem Randbereich schneidenbehaftet, so dass eine vollständig ununterbrochen umlaufende Schneide an jeder Öffnung ausgebildet ist.

Weiter bevorzugt sind alle Öffnungen jeweils geometrisch gleich ausgebildet und alle Stege jeweils geometrisch gleich ausgebildet. Hierdurch kann insbesondere eine sehr kostengünstige Herstellbarkeit sichergestellt werden.

Gemäß einer weiteren bevorzugten Ausgestaltung der Erfindung weisen die Stege eine erste Breite B1 in Umfangsrichtung auf, welche kleiner ist als eine zweite Breite B2 der Öffnungen im Umfangsrichtung. Dadurch ist es möglich, dass ein großer Bereich der Schneidenspitze durch die Öffnungen gebildet ist, so dass auf sichere Weise das komplette abgetrennte Material in den Hohlbereich des Schafts transportiert werden kann.

Besonders bevorzugt ist das distale, kuppelförmige Ende der Schneidenspitze teilkugelförmig ausgebildet.

Gemäß einer weiteren bevorzugten Ausgestaltung der Erfindung umfasst das chirurgische Instrument ferner ein Außenrohr, welches ein offenes, abgeschrägtes distales Ende aufweist, an dem die Schneidenspitze zumindest teilweise freiliegt. Das abgeschrägte Ende des Außenrohrs umgibt die Schneidenspitze somit nur in einem Teilbereich, vorzugsweise einem Viertelumfang der Schneidenspitze, so dass ein besonders guter Schutz von umgebendem Gewebe gewährleistet ist, wenn das Außenrohr, welches feststehend ist und nicht rotiert, an der entsprechenden Stelle positioniert wird.

Für einen besonders ruhigen Lauf weist das chirurgische Instrument vorzugsweise genau vier Öffnungen und genau vier Stege zwischen den Öffnungen auf. Weiter bevorzugt ist das chirurgische Instrument dabei symmetrisch zu einer Mittelachse ausgebildet.

Vorzugsweise sind die genau vier Stege derart angeordnet, dass diese bei Draufsicht auf die Spitze des chirurgischen Instruments ein Kreuz bilden. Die kreuzförmig angeordneten Stege gehen dabei besonders bevorzugt ohne Kante unmittelbare in das distale, kuppelförmige Ende der Schneidenspitze über. Weiter bevorzugt gehen die Stege ohne Kante kontinuierlich in den hohlzylindrischen Schaft des chirurgischen Instruments über. Dadurch wird eine besonders einfache Handhabbarkeit des chirurgischen Instruments erreicht. Der direkte Übergang der Stege sowohl in das kuppelförmige Ende der Schneidenspitze als auch im hohlzylindrischen Schaft ermöglicht dabei auch ein besonders sicheres Vorschieben des chirurgischen Instruments bis zu der Stelle, an welcher dann das Material entnommen werden soll. Dabei treten praktisch keine Schädigungen am Gewebe oder dergleichen bis zur Materialentnahmestelle auf.

Nachfolgend wird unter Bezugnahme auf die begleitende Zeichnung ein bevorzugtes Ausführungsbeispiel der Erfindung im Detail beschrieben. In der Zeichnung ist:
- Fig. 1: eine schematische, perspektivische Ansicht eines chirurgischen Instruments gemäß einem ersten bevorzugten Ausführungsbeispiel der Erfindung,
- Fig. 2: eine schematische, vergrößerte Seitenansicht einer Schneidenspitze des chirurgischen Instruments von Fig. 1,
- Fig. 3: eine schematische, perspektivische Ansicht aus Ansicht der Schneidenspitze des chirurgischen Instruments von Fig. 1 und
- Fig. 4: eine perspektivische Schnittansicht durch die Schneidenspitze des chirurgischen Instruments von Fig. 1.

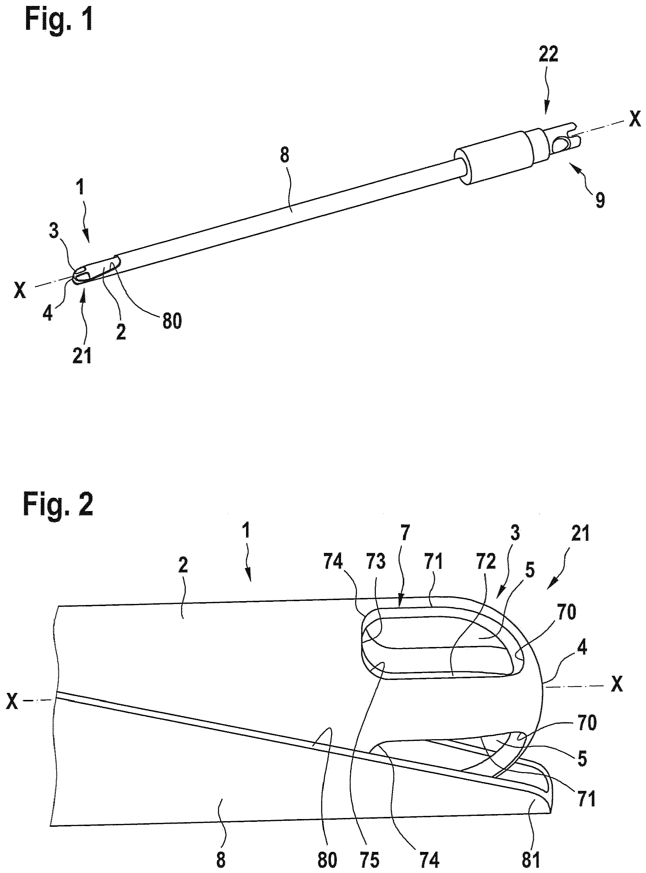

Nachfolgend wird unter Bezugnahme auf die Figuren 1 bis 4 ein chirurgisches Instrument 1 gemäß einem ersten bevorzugten Ausführungsbeispiel der Erfindung im Detail beschrieben.

Das chirurgische Instrument 1 umfasst einen Schaft 2 mit einer Schneidenspitze 3.

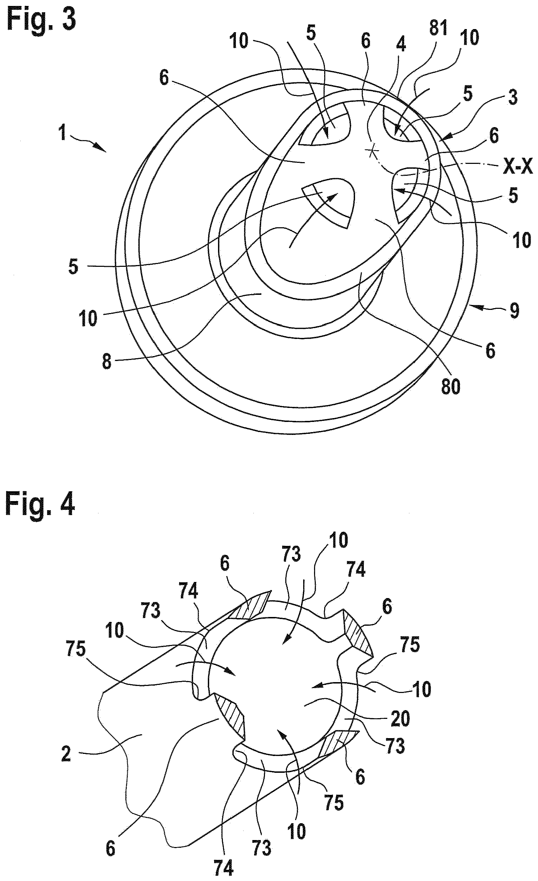

Wie insbesondere aus Fig. 4 ersichtlich ist, ist der Schaft 2 hohlzylindrisch ausgebildet und weist einen mittleren Hohlbereich 20 auf. Die Schneidenspitze 3 ist dabei an einem distalen Ende 21 des Schafts angeordnet. An einem proximalen Ende 22 des Schafts ist ein Anschluss 9 für einen Antrieb vorgesehen.

Das chirurgische Instrument 1 umfasst ferner ein Außenrohr 8, in welchem der Schaft 2 angeordnet ist. Wie aus Fig. 2 ersichtlich ist, weist das Außenrohr 8 eine schräge Öffnung 80 am distalen Ende auf. An dieser schrägen Öffnung 80 steht dann die Schneidenspitze 3 vor. Das Außenrohr 8 ist dabei nicht angetrieben und dreht sich somit nicht mit der Schneidenspitze 3 mit.

Die Schneidenspitze 3 ist im Detail aus den Figuren 2 und 4 ersichtlich. Die Schneidenspitze 3 weist hierbei genau vier Öffnungen 5 und vier Stege 6 auf. Wie aus Fig. 3 ersichtlich ist, sind die vier Stege 6 bei einer Draufsicht auf ein kuppelförmiges, schneidenfreies, distales Ende 4 der Schneidenspitze als Kreuz angeordnet. Die Schneidenspitze 3 ist dabei symmetrisch zu einer Mittelachse X-X des chirurgischen Instruments ausgebildet.

Wie insbesondere aus Fig. 2 ersichtlich ist, nehmen die Öffnungen 5 entlang des Umfangs der Schneidenspitze 3 dabei eine größere Fläche ein als die Stege 6.

Die Öffnungen 5 weisen einen ersten geradlinigen Schneidenabschnitt 71 und einen zweiten geradlinigen Schneidenabschnitt 72 auf. Der erste und zweite geradlinige Schneidenabschnitt 71, 72 liegen sich dabei an jeder Öffnung 5 einander gegenüber und sind parallel zur Mittelachse X-X. Wie aus Fig. 3 ersichtlich ist, sind der erste und zweite geradlinige Schneidenabschnitt 71, 72 über einen bogenförmigen distalen Schneidenabschnitt 70 miteinander verbunden. Ferner weist jede Öffnung einen Basisabschnitt 73 auf, welcher mit dem ersten und zweiten geradlinigen Schneidenabschnitt 71, 72 jeweils über einen ersten und zweiten bogenförmigen proximalen Schneidenabschnitt 74, 75 verbunden ist.

Somit weist jede Öffnung an ihrem Rand eine vollständig umlaufende Schneide 7 auf. Die Schneide 7 weist dabei keinerlei Unterbrechung auf und somit ist das chirurgische Instrument 1 sowohl für einen Linkslauf als auch für einen Rechtslauf geeignet. In beiden Drehrichtungen kann dabei Material von einem Knorpel oder Knochen abgetragen werden.

Wie weiter insbesondere aus Fig. 4 ersichtlich ist, vergrößern sich die Öffnungen 5 in Richtung zur Mittelachse X-X. Dadurch wird ein vereinfachtes Aufnehmen des abgetragenen Materials in den Hohlbereich 20 des Schaftes 2 möglich. In Fig. 4 ist dabei das Einströmen von abgetragenem Material durch die Pfeile 10 angedeutet.

Wie insbesondere aus Fig. 2 ersichtlich ist, kann dabei in der Position des Schafts 2 im Bereich der schrägen Öffnung 80, welche zungenartig die Schneidenspitze 3 schützt, ebenfalls noch Material in die Öffnungen 5 und damit in den Hohlbereich 20 aufgenommen werden. Die durch die schräge Öffnung 80 gebildete Zunge 81 schützt dabei an der Entnahmestelle umgebendes Gewebe, welches nicht abgetragen werden soll.

Durch die relativ großen Öffnungen 5 ergibt sich somit insbesondere für die geradlinigen Schneidenabschnitte 71, 72 ein relativ großer Abstand, wodurch sichergestellt wird, dass von dem zu entnehmenden Material relativ große Stücke abgetrennt werden können. Dadurch ergibt sich eine signifikant höhere Wahrscheinlichkeit, dass mit den abgetrennten größeren Stücken auch lebende Zellen des abzutragenden Materials abgetragen werden. Insbesondere bei den im Stand der Technik verwendeten chirurgischen Instrumenten besteht die Gefahr, dass das abzutragende Material breiförmig abgetragen wird, wobei im Material dann fast nur tote und/oder zerstörte Zellen vorhanden sind. Dadurch ist die Weiternutzung des abgetragenen Materials deutlich eingeschränkt. Bei der Erfindung dagegen kann das abzutragende Material auch in größeren Stücken mit einem hohen Anteil an unzerstörten und lebenden Zellen entnommen werden, so dass eine Weiternutzung des entnommenen Materials deutlich verbessert ist. Insbesondere kann durch das entnommene Material beispielsweise direkt ein Wiedereinbringen des entnommenen Materials an einer Stelle des Körpers, an welchem das Material benötigt wird, erfolgen. Durch die hohe Anzahl der lebenden Zellen ist dann beispielsweise ein Nachwachsen von Knorpelgewebe deutlich verbessert. Alternativ kann das mit hohem lebenden Zellanteil entnommene Material auch außerhalb des Körpers nachgezüchtet werden und dann später an entsprechend benötigter Stelle im Körper eingesetzt werden.

### Bezugszeichenliste

- 1: chirurgisches Instrument
- 2: Schaft
- 3: Schneidenspitze
- 4: kuppelförmiges, schneidenfreies Ende
- 5: Öffnungen
- 6: Stege
- 7: Schneide
- 8: Außenrohr
- 9: Anschluss für Antrieb
- 10: Strömungsrichtung des abgetrennten Materials
- 20: Hohlbereich
- 21: distales Ende des Schafts
- 22: proximales Ende des Schafts
- 70: bogenförmiger distaler Schneidenabschnitt
- 71: erster geradliniger Schneidenabschnitt
- 72: zweiter geradliniger Schneidenabschnitt
- 73: Basisabschnitt
- 74: erster bogenförmiger proximaler Schneidenbereich
- 75: zweiter bogenförmiger proximaler Schneidenbereich
- 80: schräge Öffnung
- 81: Zunge
- X-X: Mittelachse

## Patentansprüche

1. Chirurgisches Instrument zur Entnahme von Knorpelgewebe und/oder Knochenmaterial, umfassend:
- einen hohlzylindrischen Schaft (2) mit einem Hohlbereich (20) und mit einem Anschluss (9) für einen Antrieb an einem proximalen Ende (22),
- eine Schneidenspitze (3) an einem distalen Ende (21) des Schafts (2),
- wobei die Schneidenspitze (3) ein kuppelförmiges, schneidenfreies, distales Ende (4) aufweist, und
- wobei die Schneidenspitze (3) eine Vielzahl von Öffnungen (5) aufweist, welche durch eine Vielzahl von Stegen (6) voneinander getrennt sind,
- wobei an jeder Öffnung (5) an wenigstens einem durch die Stege (6) gebildeten Rand eine Schneide (7) ausgebildet ist,
**dadurch gekennzeichnet, dass**
- sich die Öffnungen (5) in Richtung zu einer Mittelachse (X-X) des chirurgischen Instruments erweitern.

2. Instrument nach Anspruch 1, wobei am Rand jeder Öffnung eine vollständige umlaufende Schneide (7) angeordnet ist.

3. Instrument nach einem der vorhergehenden Ansprüche, wobei jede Öffnung (5) einen ersten geradlinigen Schneidenabschnitt (71) an einem ersten Steg und einen zweiten geradlinigen Schneidenabschnitt (72) an einem zweiten Steg sowie einen bogenförmigen distalen Schneidenabschnitt (70), welcher die beiden geradlinigen Schneidenabschnitte (71, 72) miteinander verbindet, aufweist.

4. Instrument nach Anspruch 3, wobei jede Öffnung (5) einen zum proximalen Ende (22) des Instruments gerichteten Basisabschnitt (73), einen ersten bogenförmigen proximalen Schneidenbereich (74) und einen zweiten bogenförmigen proximalen Schneidenbereich (75) aufweist, wobei der erste und zweite bogenförmige proximale Schneidenbereich (74, 75) den ersten und zweiten geradlinigen Schneidenabschnitt (71, 72) mit dem Basisabschnitt (73) verbindet.

5. Instrument nach einem der vorhergehenden Ansprüche, wobei die Öffnungen (5) jeweils geometrisch gleich ausgebildet sind und wobei die Stege (6) jeweils geometrisch gleich ausgebildet sind.

6. Instrument nach einem der vorhergehenden Ansprüche, wobei die Stege (6) eine erste Breite in Umfangsrichtung aufweisen, welche kleiner ist als eine zweite Breite der Öffnungen (5) in Umfangsrichtung.

7. Instrument nach einem der vorhergehenden Ansprüche, wobei das distale Ende (21) der Schneidenspitze (3) teilkugelförmig ausgebildet ist.

8. Instrument nach einem der vorhergehenden Ansprüche, ferner umfassend ein Außenrohr (8), welches ein offenes, abgeschrägtes distales Ende (80) aufweist, wobei die Schneidenspitze (3) am abgeschrägten distalen Ende (80) zumindest teilweise freiliegt.

9. Instrument nach einem der vorhergehenden Ansprüche, wobei die Schneidenspitze (3) genau vier Öffnungen und genau vier Stege aufweist und die Stege (6) kreuzförmig angeordnet sind und unmittelbar in das distale Ende der Schneidenspitze (3) übergehen und/oder
wobei die Stege (6) kontinuierlich in den hohlzylindrischen Schaft (2) übergehen.

## Claims

1. A surgical instrument for removing cartilage tissue and/or bone material, comprising:
- a hollow cylindrical shaft (2) with a hollow portion (20) and with a connection (9) for a drive at a proximal end (22),
- a cutting tip (3) at a distal end (21) of the shaft (2),
- wherein the cutting tip (3) comprises a dome-shaped, non-cutting, distal end (4), and
- wherein the cutting tip (3) comprises a plurality of openings (5), which are separated from each other by a plurality of webs (6),
- wherein a cutting edge (7) is formed at each opening (5) at at least one edge formed by the webs (6),
**characterised in that**
- the openings (5) widen in direction towards a central axis (X-X) of the surgical instrument.

2. The instrument according to claim 1, wherein a complete circumferential cutting edge (7) is disposed at the edge of each opening.

3. The instrument according to one of the preceding claims, wherein each opening (5) comprises a first rectilinear cutting edge portion (71) at a first web and a second rectilinear cutting edge portion (72) at a second web, and an arcuate distal cutting edge portion (70), which connects the two rectilinear cutting edge portions (71, 72) to each other.

4. The instrument according to claim 3, wherein each opening (5) comprises a base portion (73) directed towards a proximal end (22) of the instrument, a first arcuate proximal cutting portion (74) and a second arcuate proximal cutting portion (75), wherein the first and second arcuate proximal cutting portions (74, 75) connect the first and second rectilinear cutting portions (71, 72) with the base portion (73).

5. The instrument according to one of the preceding claims, wherein the openings (5) are each geometrically formed in the same manner, and wherein the webs (6) are each geometrically formed in the same manner.

6. The instrument according to one of the preceding claims, wherein the webs (6) comprise a first width in the circumferential direction, which is smaller than a second width of the openings (5) in circumferential direction.

7. The instrument according to one of the preceding claims, wherein the distal end (21) of the cutting tip (3) is formed to be part-spherical.

8. The instrument according to any of the preceding claims, further comprising an outer tube (8), which comprises an open, bevelled distal end (80), wherein the cutting tip (3) is at least partially exposed at the bevelled distal end (80).

9. The instrument according to one of the preceding claims, wherein the cutting tip (3) comprises exactly four openings and exactly four webs, and the webs (6) are disposed in a cross shape and merge directly into the distal end of the cutting tip (3), and/or
wherein the webs (6) continuously merge into the hollow cylindrical shaft (2).

## Revendications

1. Instrument chirurgical destiné à l'enlèvement de tissu cartilagineux et/ou de matière osseuse, comprenant :
- un manche cylindrique creux (2) avec une zone creuse (20) et avec un raccord (9) pour un entraînement sur une extrémité proximale (22),
- une pointe coupante (3) sur une extrémité distale (21) de l'arbre (2),
- dans lequel la pointe coupante (3) présente une extrémité distale (4) en forme de dôme sans tranchant, et
- dans lequel la pointe coupante (3) présente une pluralité d'ouvertures (5), lesquelles sont séparées les unes des autres par une pluralité d'entretoises (6),
- dans lequel un tranchant (7) est réalisé sur chaque ouverture (5) sur au moins un bord formé par les entretoises (6),
**caractérisé en ce que**
- les ouvertures (5) s'élargissent en direction d'un axe central (X-X) de l'instrument chirurgical.

2. Instrument selon la revendication 1, dans lequel un tranchant (7) totalement périphérique est disposé sur le bord de chaque ouverture.

3. Instrument selon l'une quelconque des revendications précédentes, dans lequel chaque ouverture (5) présente une première section tranchante rectiligne (71) sur une première entretoise et une deuxième section tranchante rectiligne (72) sur une deuxième entretoise ainsi qu'une section tranchante distale en forme d'arc (70), laquelle relie entre elles les deux sections tranchantes (71, 72) rectilignes.

4. Instrument selon la revendication 3, dans lequel chaque ouverture (5) présente une section de base (73) dirigée vers l'extrémité proximale (22) de l'instrument, une première zone tranchante proximale (74) en forme d'arc et une deuxième zone tranchante proximale (75) en forme d'arc, dans lequel la première et la deuxième zone tranchante (74, 75) proximale en forme d'arc relient la première et la deuxième section tranchante rectiligne (71, 72) à la section de base (73).

5. Instrument selon l'une quelconque des revendications précédentes, dans lequel les ouvertures (5) sont réalisées respectivement de manière identique sur le plan géométrique et dans lequel les entretoises (6) sont réalisées respectivement de manière identique sur le plan géométrique.

6. Instrument selon l'une quelconque des revendications précédentes, dans lequel les entretoises (6) présentent une première largeur dans la direction périphérique, laquelle est inférieure à une deuxième largeur des ouvertures (5) dans la direction périphérique.

7. Instrument selon l'une quelconque des revendications précédentes, dans lequel l'extrémité distale (21) de la pointe coupante (3) est réalisée en forme de sphère partielle.

8. Instrument selon l'une quelconque des revendications précédentes, comprenant en outre un tube extérieur (8), lequel présente une extrémité distale chanfreinée (80) ouverte, dans lequel la pointe coupante (3) est au moins en partie exposée sur l'extrémité distale chanfreinée (80).

9. Instrument selon l'une quelconque des revendications précédentes, dans lequel la pointe coupante (3) présente précisément quatre ouvertures et précisément quatre entretoises, et les entretoises (6) sont disposées en forme de croix et se confondent directement avec l'extrémité distale de la pointe coupante (3), et/ou
dans lequel les entretoises (6) se confondent en continu avec l'arbre (2) cylindrique creux.
